# FASCICULE DE BREVET EUROPEEN

(11) **EP 2 081 948 B1**
(45) Date de publication et mention de la délivrance du brevet: **09.03.2011**
(21) Numéro de dépôt: 07823852.4
(22) Date de dépôt: 26.09.2007
(51) Int. Cl.: C07H 21/00, C12Q 1/68, G01N 21/64

(54) **NOUVEL OLIGONUCLEOTIDE MARQUE**
NEUES MARKIERTES OLIGONUKLEOTID
NOVEL LABELLED OLIGONUCLEOTIDE

(30) Priorité: 28.09.2006 FR 0653982
(43) Date de publication de la demande: 29.07.2009
(73) Titulaire: bioMérieux S.A., 69280 Marcy l'Etoile (FR)
(72) Inventeur: BERNAL-MENDEZ, Eloy, 38070 Saint-Quentin-Fallavier (FR); LAAYOUN, Ali, 38690 Colombe (FR); LAURENT, Alain, 38100 Grenoble (FR)
(86) Numéro de dépôt international: PCT/FR2007/052007
(87) Numéro de publication internationale: WO 2008/037924

(56) Documents cités:
- WO-A-88/04301
- WO-A-90/15813
- US-A- 5 866 336
- US-A- 6 117 635
- TYAGI, SANJAY ET AL: "Molecular beacons : probes that fluoresce upon hybridization" NATURE BIOTECHNOLOGY , 14(3), 303 -8 CODEN: NABIF9; ISSN: 1087-0156, 1996, XP002427012 cité dans la demande

### SEQUENCE LISTING

<110> bioMérieux SA
<120> Nouvel oligonucleotide marque
<130> Unknown
<160> 18
<170> Patent In version 3.3
<210> 1
   <211> 30
   <212> DNA
   <213> Human metapneumovirus
<400> 1
   catataagca tgctatatta aaagagtctc 30
<210> 2
   <211> 28
   <212> DNA
   <213> Human metapneumovirus
<400> 2
   cctatttctg cagcatattt gtaatcag 28
<210> 3
   <211> 38
   <212> DNA
   <213> Human metapneumovirus
<400> 3
   gctaccaact gcagtgacac cctcatcatt gcagtagc 38
<210> 4
   <211> 38
   <212> DNA
   <213> Human metapneumovirus
<400> 4
   gctacacgtt actactccca cagtgacgtc aacgtagc 38
<210> 5
   <211> 38
   <212> DNA
   <213> Human metapneumovirus
<400> 5
   gctacacgtt actactccca cagtgacgtc aacgtagc 38
<210> 6
   <211> 38
   <212> DNA
   <213> Human metapneumovirus
<400> 6
   gctaccaact gcagtgacac cctcatcatt gcagtagc 38
<210> 7
   <211> 33
   <212> DNA
   <213> Human metapneumovirus
<400> 7
   acgttactac tcccacagtg acgtcaacgt agc 33
<210> 8
   <211> 27
   <212> DNA
   <213> Human metapneumovirus
<400> 8
   tgcaatgatg agggtgtcac tgcggtt 27
<210> 9
   <211> 38
   <212> DNA
   <213> Human metapneumovirus
<400> 9
   cgatgcaact gcagtgacac cctcatcatt gcacatcg 38
<210> 10
   <211> 38
   <212> DNA
   <213> Human metapneumovirus
<400> 10
   cgatgcaact gcagtgacac cctcatcatt gcacatcg 38
<210> 11
   <211> 38
   <212> DNA
   <213> Human metapneumovirus
<400> 11
   gctacacgt actactccca cagt gacgt c aac gt agc 38
<210> 12
   <211> 38
   <212> DNA
   <213> Human metapneumovirus
<400> 12
   gctacacgtt actactccca cagtgacgtc aacgtacg 38
<210> 13
   <211> 24
   <212> DNA
   <213> Human metapneumovirus
<400> 13
   ttatgatgag ggtgtcactg catt t 24
<210> 14
   <211> 24
   <212> DNA
   <213> Human metapneumovirus
<400> 14
   ttatgatgag gctgtcact catt 24
<210> 15
   <211> 24
   <212> DNA
   <213> Human metapneumovirus
<400> 15
   ttatgatgag gttgtcactg catt 24
<210> 16
   <211> 24
   <212> DNA
   <213> Human metapneumovirus
<400> 16
   ttatgatgag gatgtcactg catt 24
<210> 17
   <211> 31
   <212> DNA
   <213> Human metapneumovirus
<400> 17
   cgatgtgcaa tgatgagggt gtcactgcat t 31
<210> 18
   <211> 31
   <212> DNA
   <213> Human metapneumovirus
<400> 18
   gtagctgcaa tgatgagggt gtcactgcat t 31

## Description

La présente invention concerne un nouvel oligonucléotide marqué. L'invention concerne également un procédé mettant en oeuvre un tel oligonucléotide.

Il est souvent nécessaire, dans les technologies relatives aux acides nucléiques et au matériel génétique, de déterminer si un gène, une partie de gène ou une séquence nucléotidique est présent dans un organisme vivant, un extrait cellulaire de cet organisme ou tout autre un échantillon biologique.

Différents types de méthodes de détection des acides nucléiques sont décrits dans la littérature. Ces méthodes, et particulièrement celles qui requièrent la détection de polynucléotides, reposent sur les propriétés d'appariement des brins complémentaires d'acides nucléiques, communément appelée "hybridation d'acides nucléiques" ou simplement "hybridation".

D'une manière générale, après avoir identifié la séquence spécifique d'un organisme ou d'une maladie qui doit être analysée, il convient d'extraire les acides nucléiques d'un échantillon, d'amplifier et de détecter la séquence d'intérêt. De nombreuses méthodes d'amplification et de détection ont été développées dans ce but.

Ainsi, la PCR (Polymerase Chain Réaction) est basée sur la répétition d'un processus en trois étapes : la dénaturation de l'ADN double brin, l'hybridation des amorces à l'ADN simple brin, et l'extension enzymatique des amorces par une ADN polymérase thermostable qui synthétise un brin d'ADN complémentaire à celui servant de cible pour les amorces oligonucléotidiques.

L'amplification peut être analysée en fin de cycle (PCR « end point ») ou en temps réel (PCR en temps réel) grâce à l'utilisation d'un marquage fluorescent du produit amplifié. Il existe plusieurs techniques de PCR en temps réel. L'une d'elles met en oeuvre des sondes particulières appelées « molecular beacons », qui sont des séquences en épingle à cheveux (ou « hairpin ») comprenant une structure boucle « loop » , une tige « stem« contenant un fluorophore à une extrémité de la séquence et un extincteur de fluorescence (ou « quencher ») à l'autre extrémité (Tyagi, S. and Kramer, FD., Nat. Biotechnol., 1996, 14, 303-308, Marras, SA. et al., Genet Anal., 1999, 14, 151-156). En dessous de la température d'hybridation, ces séquences, non hybridées à une séquence cible, adoptent une configuration dite en " épingle à cheveux " : les extrémités 5' et 3' sont proches, il n'y a pas d'émission de fluorescence. Lorsque la sonde via la séquence de la boucle (loop) reconnaît et s'hybride à un amplicon, qui correspond à la séquence cible amplifiée, la structure tige (stem) est déstabilisée, les extrémités sont éloignées, le "quencher" ne joue plus son rôle et il y a émission de fluorescence.

Les documents US-A5,866,336 et US-A-6,117,635, des mêmes inventeurs, proposent également des sondes dites « molecular beacons », également appelées balises moléculaires, mais dans une configuration particulière. Ainsi l'extrémité 3' de la sonde se prolonge sous la forme d'une amorce d'amplification. Cette dernière, une fois hybridée à sa cible complémentaire, permet par élongation, et éventuellement avec l'aide d'une amorce d'amplification adaptée, l'amplification de la cible.

Toutefois cette configuration oblige les scientifiques à trouver non seulement deux zones adjacentes pour définir l'amorce d'amplification (en général choisie pour leur sensibilité) et la sonde de détection (en général choisie pour leur spécificité). La définition de telles zones même en l'absence de la contrainte de voisinage étant déjà difficile, le challenge est dans ce cas bien plus ardu voir dans certains cas impossible.

Toutefois, dans certaines conditions, l'émission de fluorescence par des sondes « molecular beacons » peut être « parasitée » par un bruit de fond dû à l'éloignement du fluorophore et du quencher suite à une dégradation par des nucléases présentes lors de la réaction d'amplification. C'est notamment le cas lors d'une PCR « classique », puisque l'enzyme Taq polymérase elle même possède une activité 5' nucléase. De plus, outre l'émission de fluorescence « parasite » qui apparaît lors d'une PCR en temps réel, le clivage des « molecular beacons » du à la présence résiduelle d'enzyme thermostable polymerase en fin de cycle affecte une analyse de gradient de température postérieure (« end point ») à l'amplification. Or cette analyse de gradient de température postérieure à l'amplification est la méthode la plus sensible pour détecter des variations simples des séquences, comme les SNP ("single nucleotide polymorphisme », ou polymorphisme d'un seul nucléotide) par les « molecular beacons ».

La NASBA est une autre technologie d'amplification, isotherme, de l'acide nucléique reposant sur l'action conjointe de trois enzymes (transcriptase inverse AMV, RNAseH et polymérase-ARN T7). L'amplification peut être analysée en fin de cycle (NASBA « end point ») ou en temps réel (NASBA en temps réel) grâce à l'utilisation d'un marquage fluorescent du produit amplifié, par l'utilisation de sondes « molecular beacons ». Toutefois, en « end point », la présence de RNAse H, enzyme indispensable lors de l'initiation de la NASBA, peut induire, en fin de réaction, une dégradation de l'ARN cible hybridé sur le « molecular beacon ». On observe alors une diminution du signal de fluorescence qui est due au clivage de l'ARN cible. Une analyse de gradient de température postérieure à l'amplification est donc délicate.

Que ce soit en PCR ou en NASBA , l'émission de fluorescence peut également être parasitée par l'hybridation non spécifique de la partie tige du «molecular beacon » sur l'amplicon. Il est alors important de réduire cette hybridation parasite.

Enfin, les « molecular beacons » peuvent être également utilisés pour la détection de séquences cibles indépendamment d'une amplification. En effet, il est possible d'analyser l'expression d'un gène dans des cellules vivantes par l'injection de «molecular beacons», la fluorescence émise reflétant l'hybridation de la sonde sur un transcrit cible. Toutefois, la présence de nombreuses nucléases en activité dans la cellule implique une forte dégradation des sondes, et donc induit l'émission de fluorescence qui n'est pas due à l'hybridation de la sonde sur une séquence cible.

Il est donc important d'améliorer les techniques d'amplification actuelles afin de les rendre plus sensibles, en diminuant notamment les phénomènes de clivage et d'hybridation non spécifiques des « molecular beacons » de l'état de la technique.

La présente invention se propose de résoudre l'ensemble des inconvénients de l'état de la technique en présentant de nouveaux oligonucléotides marqués spécifiques, stables et résistants aux nucléases.

A ce titre, l'invention concerne un oligonucléotide marqué comprenant un premier segment nucléotidique, un deuxième segment nucléotidique, complémentaire d'une séquence cible, et un troisième segment nucléotidique, complémentaire dudit premier segment nucléotidique caractérisé en ce qu'il comprend un fluorophore, un extincteur et au moins un nucléoside d'anomérie alpha.

Préférentiellement, lesdits premier et troisième segments sont de part et d'autre du deuxième segment.

Préférentiellement, ledit deuxieme segment est constitué de nucléosides d'anomerie alpha.

Préférentiellement, l'oligonucléotide marqué selon l'invention est caractérisé en ce que le fluorophore est à une extrémité dudit oligonucléotide et l'extincteur est à l'autre extrémité dudit oligonucléotide.

Préférentiellement, ledit fluorophore est une fluoresceine. Préférentiellement, ledit extincteur est le dabsyl.

Préférentiellement, ledit premier segment comprend de 3 à 8 nucléotides, ledit deuxième segment comprend de 10 à 35 nucléotides, et ledit troisième segment, lorsqu'il est présent comprend de 3 à 8 nucléotides.

L'invention concerne également un procédé pour détecter un matériel nucléique dans un échantillon biologique comprenant les étapes suivantes :
a) on extrait le matériel nucléique d'un échantillon biologique,
b) on amplifie le matériel nucléique pour obtenir des amplicons d'au moins une séquence cible du matériel nucléique
c) on utilise, simultanément à l'étape b) ou postérieurement à l'étape b), au moins un oligonucléotide marqué selon l'invention
d) on détecte la présence desdits amplicons

Selon un mode préféré de réalisation de l'invention, on amplifie le matériel nucléique par PCR

Selon un mode préféré de réalisation de l'invention, on amplifie le matériel nucléique par PCR.

Selon un autre mode préféré de réalisation de l'invention, on amplifie le matériel nucléique par NASBA.

Les définitions suivantes permettront de mieux comprendre l'invention.

Au sens de la présente invention, on entend par «amont », une région située du coté de l'extrémité 5' de l'acide nucléique ou de la séquence polynucléotidique, et par « aval » une région située du coté de l'extrémité 3' dudit acide nucléique ou de ladite région polynucléotidique.

Les termes fragment nucléotidique, fragments d'acide nucléique, segment nucléotidique. segment d'acide nucléique, séquences nucléotidique, séquence d'acide nucléique, ou olignucléotide, désignent un fragment d'ADN ou d'ARN naturel, un polynucléotide naturel ou de synthèse, un fragment d'ADN ou d'ARN de synthèse non modifiée ou comprenant au moins une base modifiée telles que l'inosine, la méthyl-5-désoxycitidine, la diméthylamino-5-desoxyuridine, la désoxyuridine, la diamino 2,6 purine, la bromo 5 désoxyuridine, la pseudouridine, la pseudoisocytidine, ou toute autre base modifiée permettant l'hybridation. Chacune des modifications peut être prise en combinaison.

Par oligonucléotide marqué, ou sonde, on entend un enchaînement de nucléosides, comprenant en outre au moins une molécule permettant la détection dudit oligonucléotide.

Cet oligonucléotide peut être notamment une sonde de détection agissant par reconnaissance moléculaire. Par sonde de détection agissant par reconnaissance moléculaire, on entend une séquence nucléique de 10 à 100 motifs nucléotidiques, notamment de 15 à 45 motifs nucléotidiques, possédant une spécificité d'hybridation dans des conditions déterminés pour former un complexe d'hybridation avec une séquence nucléique cible et émettant un signal lors de l'hybridation de la sonde sur la séquence nucléique cible.

La sonde de détection peut être notamment une sonde de détection « molecular beacon » telle que décrite par Tyagi & Kramer (Nature Biotech, 1996, 14:303-308). Ces « molecular beacons » deviennent fluorescentes lors de l'hybridation. Elles possèdent une structure de type tige-boucle et contiennent un fluorophore et un groupe « quencher ». La fixation de la séquence de boucle spécifique avec sa séquence complémentaire d'acide nucléique cible provoque un déroulement de la tige et l'émission d'un signal fluorescent lors de l'excitation à la longueur d'onde qui convient.

La sonde de détection peut être notamment une sonde intégrée dans des amorces ou primers du type « Scorpion® » (Nucleic Acids Research, 2000, Vol. 28, No. 19 3752-3761) ou « Amplifluor ® » (Genome Research Vol 1 163-169, 2001 Myakishev M. *et al).*

Les Amplifluor® sont des séquences nucléotidiques qui possèdent du côté 5' une séquence avec une structure tige-boucle, avec un fluorophore à l'extrémité 5', et un "quencher" de type Dabsyl à l'autre extrémité de la structure. Du côté 3', les Amplifluor® possèdent une séquence qui est complémentaire d'une séquence de la cible et qui agit en tant qu'amorce lors de la réaction d'amplification.

Les Scorpions® sont des séquences qui possèdent du côté 5' une séquence avec une structure tige-boucle avec un "quencher" à une extrémité de la dite structure et un fluorophore à l'autre extrémité de la dite structure. Du côté 3', les Scorpions® possèdent une séquence qui est complémentaire d'une séquence de la cible et qui agit en tant qu'amorce lors de la réaction d'amplification. Dans le mode classique des Scorpions, un « espaceur », situé entre la séquence amorce et la séquence tige-boucle permet d'empêcher la reconnaissance de la séquence tige-boucle par la polymerase. Cet espaceur devient optionnel si les Scorpions sont réalisés selon l'invention.

Au sens de la présente invention, on entend par « fluorophore » une molécule qui émet un signal de fluorescence entre 500 et 700nm quand elle est excitée par de la lumière à une longueur d'onde qui convient (ou entre 450 et 650 nm). Le fluorophore peut être notamment une rhodamine ou un dérivé tel que Texas Red, une fluorescéine ou un dérivé, Au sens de la présente invention, on entend par « fluorescéine » une molécule chimique aromatique qui émet un signal de fluorescence avec un maximum d'émission autour de 530 nm, quand elle est excitée par de la lumière à une longueur d'onde autour de 495 nm.

Au sens de la présente invention, on entend par « extincteur » (ou ""quencher"") une molécule qui interfère avec la fluorescence émise par un fluorophore. Cet extincteur est notamment choisi parmi des molécules aromatiques non fluorescentes, pour éviter des émissions parasites. Préférentiellement, le dit « extincteur » est un Dabsyl ou un Dabcyl ou un « Black hole quencher™ ». Le Dabcyl, le Dabsyl et les « Black hole quencher™ » sont des molécules aromatiques non fluorescentes qui empêchent l'émission de fluorescence quand elles se trouvent physiquement à proximité d'un fluorophore, ou par FRET.

Par « nucléoside d'anomérie alpha » ou alpha nucléosides, ou nucléosides alpha, on entend des déoxynucléosides à configuration anomérique non naturelle alpha, dans lequel la base azotée portée par le carbone anomérique du deoxyribose est située en dessous du plan au lieu d'être en dessus du plan comme dans le cas des nucléosides beta. Préférentiellement, les nucléotides alpha sont ceux décrits dans la demande WO 88/04301 et WO-A-90/15813, des mêmes inventeurs. Dans ces documents il est établi que les oligonucleotides α manifestent une résistance très élevée aux enzymes par rapport aux oligonucléotides β ou naturels.

Toutefois ces deux documents ne proposent que des oligonucléotides dont tous les nucléotides sont constitués de nucléotides α, pour lesquels il n'est pas impossible d'extrapoler une résistance d'oligonucléotides α vers un oligonucléotide pouvant n'avoir qu'un nucléotide α.

D'autre part, la structure en épingle à cheveux est une structure complexe ou également appelée « balise moléculaire » dans laquelle un équilibre existe entre la forme fermée (forme en épingle à cheveu, absence de la cible et pas de détection) et la forme ouverte (hybride sur la cible et détection).

Pour que ceci soit réalisé, il faut que des conditions d'auto-hybridation et d'hybridation (Tm notamment) soient réunies.

Celles-ci sont bien connues pour des oligonucléotides classiques mais pas pour des balises moléculaires surtout si elles incorporent des nucléotide d'anomérie α. De ce fait, la réalisation d'une balise moléculaire ayant un ou plusieurs nucléotides d'anomérie α n'est absolument pas évident pour l'homme du métier.

Par « extrémité », on entend le point de départ et le point de terminaison de la synthèse d'un oligonucléotide généralement définis par le numéro porté par les hydroxyles libres portés par le premier ou le dernier nucléoside soit 3' ou 5'. Il est entendu que par un choix approprié des unités d'élongation (les phosphoramidites des nucléosides alpha ou beta), on peut synthétiser un oligonucléotide dans le sens 3' vers 5' ou l'inverse et voire alterner pendant la synthèse le sens de l'élongation. Ceci conduit à des oligonucléotides portant des extrémités 3'-5, 5'-3' ou 3'-3', 5'-5'.

Par « premier segment », on entend une séquence nucléotidique qui peut être complémentaire et de polarité adaptée audit troisième segment lors de la présence de ce dernier.

Par «deuxième segment », on entend une séquence nucléotidique complémentaire et de polarité adaptée à la séquence de la cible.

Par «troisième segment », on entend une séquence nucléotidique complémentaire et de polarité adaptée audit premier segment.

On parle également de parties «tige » pour lesdits premier et troisième segment, et de partie «boucle » pour ledit deuxième segment. Ce mode de réalisation particulier permet d'obtenir un oligonucléotide marqué de type « molecular beacons ».

Par séquence ou région capable de s'hybrider sur une autre séquence/région, ou séquence complémentaire, on entend une séquence ou une région pouvant s'hybrider sur une autre séquence/région dans des conditions d'hybridation, qui peuvent être déterminées dans chaque cas de façon connue. On parle également de séquences/régions complémentaires. Une séquence ou région strictement complémentaire d'une autre est une séquence dans laquelle chacune des bases peut s'apparier avec une base de l'autre séquence, sans mésappariement. Par hybridation, on entend le processus au cours duquel, dans des conditions appropriées, deux fragments nucléotidiques, ayant des séquences suffisamment complémentaires sont susceptibles de former un double brin avec des liaisons hydrogènes stables et spécifiques. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

Par « polarité », on entend l'orientation de la séquence nucléotidique, 5' vers 3' ou 3' vers 5', par rapport à sa séquence complémentaire. Ainsi des segments peuvent être orientés de façon :
□ antiparallèle : c'est le cas d'un oligonucléotide de configuration naturelle 5 et de sa cible de configuration naturelle β? ou c'est le cas d'un oligonucléotide de configuration non naturelle α et de sa cible de configuration non naturelle α?
□ parallèle : c'est le cas d'un oligonucléotide de configuration non naturelle α et de sa cible de configuration naturelle β?En fonction de ces règles de polarité, on peut obtenir des oligonucléotides 5'-3', 3'-3' ou 5'-5' qui sont synthétisés par l'homme du métier à partir de molécules adaptées.

Par « enzyme polymérase », on entend une enzyme capable de synthétiser un fragment d'ADN ou d'ARN complémentaire à partir d'une matrice d'acide nucléique et d'un oligonucléotide initiateur (ou primer). Les enzymes polymérase ont parfois une activité nucléase qui conduit à la dégradation des fragments d'acides nucléiques qui s'hybrident sur la cible en cours de copie : bloquer cette activité est alors très pertinent dans une approche en temps réel car le signal enregistré est dû uniquement à la reconnaissance moléculaire et non à une dégradation enzymatique.

Au sens de la présente invention, on entend par « matériel nucléique », une séquence d'acides nucléiques telle que une séquence d'acides désoxyribonucléiques (ADN) ou d'acides ribonucléiques (ARN). Selon un mode préféré de réalisation de l'invention, le matériel nucléique comprend une séquence d'acides désoxyribonucléiques. Selon un mode préféré de réalisation de l'invention, le matériel nucléique est extrait d'un échantillon biologique prélevé chez un patient.

Au sens de la présente invention, on entend par « échantillon biologique », tout échantillon susceptible de contenir un matériel nucléique tel que défini ci après. Cet échantillon biologique peut être prélevé chez un patient et peut être notamment un échantillon de tissus, de sang, de sérum, de salive, de cellules circulantes du patient. Cet échantillon peut également être un échantillon alimentaire. On dispose de cet échantillon biologique par tout type de prélèvement connu de l'homme du métier.

Au sens de la présente invention, on entend par « séquence cible », une séquence nucléotidique dont au moins une partie de l'enchaînement en motifs nucléotidiques est spécifique et complémentaire de la séquence nucléotidique de la sonde de détection utilisée.

Au sens de la présente invention, lors de l'étape a) on extrait le matériel nucléique d'un échantillon biologique par tout protocole connu de l'homme du métier. A titre indicatif, l'extraction d'acides nucléiques peut être réalisée par une étape de lyse des cellules présentes dans l'échantillon biologique, afin de libérer les acides nucléiques contenus dans les enveloppes protéiques et/ou lipidiques des cellules (comme des débris cellulaires qui perturbent les réactions ultérieures). A titre d'exemple, on peut utiliser les méthodes de lyse telles que décrite dans les demandes de brevet WO 00/05338 sur la lyse mixte magnétique et mécanique, WO 99/53304 sur la lyse électrique, et WO 99/15321 sur la lyse mécanique.

L'homme du métier pourra utiliser d'autres méthodes de lyse bien connues, telles que les chocs thermiques ou osmotiques ou les lyses chimiques par des agents chaotropiques tels que les sels de guanidium (US 5,234,809). Cette étape de lyse peut également être suivie d'une étape de purification, permettant la séparation entre les acides nucléiques et les autres constituants cellulaires relargués dans l'étape de lyse. Cette étape permet généralement de concentrer les acides nucléiques, et peut être adapté à la purification d'ADN ou d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétique, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO 97/45202 et WO 99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep^{®} Silica, Promega: MagneSil™ Paramagnetic particles). D'autres méthodes très répandues reposent sur des résines échangeuses d'ions en colonne ou en format particulaire paramagnétique (Whatman: DEAE-Agarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind^{™}).

Lorsque l'on souhaite extraire spécifiquement l'ADN d'un échantillon biologique, on peut notamment réaliser une extraction par du phénol, du chloroforme et de l'alcool pour éliminer les protéines et précipiter l'ADN avec de l'alcool 70%. L'ADN peut alors être culoté par centrifugation, lavé et remis en suspension.

Au sens de la présente invention, l'« étape b) » est un processus vénérant de multiples copies (ou amplicons) d'une séquence nucléique par l'action d'au moins une enzyme polymérase. Au sens de la présente invention, on entend par amplicons les copies de la séquence cible obtenues lors d'une réaction d'amplification enzymatique.

Selon un mode préféré de réalisation de l'invention, on amplifie le matériel nucléique par PCR et l'étape b) est une succession de cycles comprenant les étapes suivantes :
o la dénaturation de la séquence cible afin d'obtenir deux brins cibles d'ADN complémentaires, ou de destructurer le brin cible d'ARN,
o l'hybridation de chacun des brins cibles, obtenus lors de l'étape de dénaturation précédente avec au moins une amorce d'amplification ,
o la formation à partir des amorces d'amplification des brins complémentaires aux brins sur lesquels elles sont hybridées en présence d'une enzyme polymérase et de nucléosides triphosphate
ce cycle étant répété un nombre de fois déterminé pour obtenir la séquence cible dans une concentration suffisante pour permettre sa détection.

Selon ce mode de réalisation, les étapes b) et c) sont effectuées en même temps ou l'une après l'autre.

Lorsque les étapes b) et c) sont effectuées en même temps, ce mode de réalisation est préférentiellement mise en oeuvre par «PCR en temps réel » qui regroupe en une étape unique la technique d'amplification PCR et la détection, et qui fait appel notamment à des « molecular beacons ». La réaction PCR intervient dans le tube, produisant des amplicons avec lequel les «molecular beacons » spécifiques peuvent s'hybrider pour donner un signal fluorescent. La formation des nouvelles molécules d'ADN est mesurée en temps réel par contrôle du signal dans un lecteur fluorescent, pendant l'étape d'hybridation. L'utilisation d'oligonucléotides marqués selon la présente invention permet d'éviter que les sondes de détection soient dégradées par l'enzyme d'amplification (par exemple la Taq Polymérase), ce qui augmente la sensibilité de la détection et améliore les performances de la technique. Lorsque les étapes b) et c) sont effectuées l'une après l'autre, la réaction PCR intervient dans le tube, produisant des amplicons. A la fin de cette étape d'amplification, les « molecular beacons » sont ajoutés au milieu de réaction, et peuvent s'hybrider pour donner un signal fluorescent. L'utilisation d'oligonucléotides marqués selon la présente invention permet d'éviter que les sondes de détection soient dégradées par l'enzyme polymérase thermostable d'amplification, qui reste de manière résiduelle dans le tube de réaction, ce qui augmente la sensibilité de la détection et améliore les performances de la technique.

Selon un autre mode préféré de réalisation de l'invention, lors de l'étape b), on amplifie le matériel nucléique par NASBA.

Selon ce mode de réalisation, les étapes b) et c) sont préférentiellement effectuées l'une après l'autre. A la fin de l'étape d'amplification, les « molecular beacons » sont ajoutés au milieu de réaction, et peuvent s'hybrider pour donner un signal fluorescent. L'utilisation d'oligonucléotides marqués selon la présente invention permet d'éviter que les sondes de détection soient dégradées par l'enzyme RNAseH, qui reste de manière résiduelle dans le tube de réaction, ce qui augmente la sensibilité de la détection et améliore les performances de la technique.

L' « étape d) » est réalisée par la détection du signal de fluorescence émis lors de l'hybridation de l'oligonucléotide marqué selon l'invention sur l'amplicon, et peut être réalisée par tous protocoles connus de l'homme du métier.

Les figures ci-jointes sont données à titre d'exemple explicatif et n'ont aucun caractère limitatif. Elles permettront de mieux comprendre l'invention.
La figure 1 présente un exemple d'oligonucléotide marqué selon l'invention comportant un premier segment et un troisième segment (tige) synthétisés avec des nucléosides alpha (en grisé) et un deuxième segment (boucle) synthétisé avec des nucléosides beta tel que décrit dans l'exemple 1A.
La figure 2 présente un exemple d'oligonucléotide marqué selon l'invention comportant un premier segment et un troisième segment (tige) synthétisés avec des nucléosides beta et un deuxieme segment (boucle) synthétisé avec des nucléosides alpha (en Grisé) tel que décrit dans l'exemple 1B.
La figure 3 présente un exemple d'oligonucléotide marqué selon l'invention comportant un premier segment et un troisième segment (tige), ainsi qu'un deuxième segment (boucle) synthétisés avec des nucléosides alpha (en grisé) tel que décrit dans l'exemple 1C.

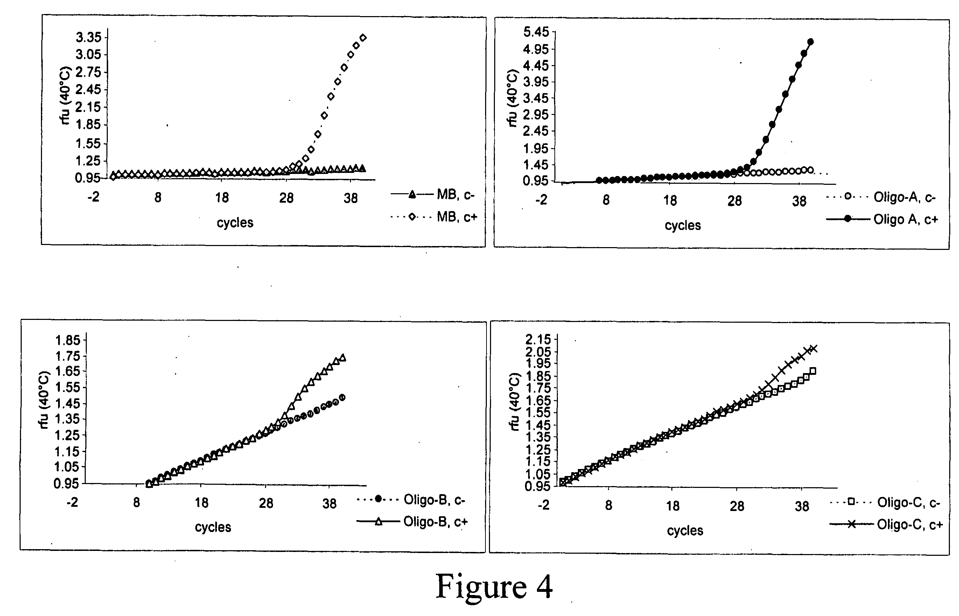

La figure 4 présente les profils de fluorescence normalisés mesurés à 40°C des molecular beacons modifiés ou non tels que décrit dans l'exemple 2A.
La figure 5 présente les profils de fluorescence normalisés mesurés à 95°C pour les molecular beacons « Oligonucléotide A » et « MB » pendant l'amplification sur Lightcycler tels que décrit dans l'exemple 2A.
La figure 6 présente les profils de fluorescence normalisés mesurés à 95°C pour les molecular beacons « Oligonucléotide B » et « MB » pendant l'amplification sur Lightcycler tels que décrit dans l'exemple 2A.
La figure 7 présente les profils de fluorescence normalisées mesurés à 95°C pour les molecular beacons «Oligonucléotide C » et « MB » pendant l'amplification sur Lightcycler tels que décrit dans l'exemple 2A.
La figure 8 présente les profils de dénaturation thermique avec mesure de la fluorescence pour les sondes étudiées avec les différentes séquences complémentaires avec ou sans mismatchs, tel que décrit dans l'exemple 3.
La figure 9 présente les profils de dénaturation thermique avec mesure de la fluorescence pour les sondes étudiées avec les cibles complémentaires ou pas d'une partie de la tige, tel que décrit dans l'exemple 4.

Les exemples suivants sont donnés à titre illustratif et n'ont aucun caractère limitatif Ils permettront de mieux comprendre l'invention.

### Exemple 1 : Procédé de synthèse des oligonucléotides selon l'invention

***Matières premières** :* Les nucléotides naturels qui ont été modifiés en 3' par un groupement phosphoramidite sont appelés 3'amidites-beta. Ces oligonucléotides sont commerciaux et achetés chez Glen Research (Foster City, USA).

Les nucléotides naturels qui ont été modifiés en 5' par un groupement phosphoramidite sont appelés 5'amidites-beta, ils sont également commerciaux et achetés chez Glen Research (Foster City, USA).

Les nucléotides d'anomérie alpha modifiés en 3' par un groupement phosphoramidite sont appelés 3'amidites-alpha, ils sont commerciaux et achetés chez Chemgenes (Wilmington, USA).

La CPG dabsyl (Controled pore glass, CPG) et le phosphoramidite de la 6-Fluoresceine sont commercialisés par Glen Research (Foster City, USA).

**TABLEAU 1**

| Molécules | Nom | Ref. |
|---|---|---|
| 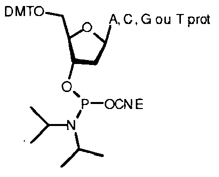 | 3'amidites-beta | Glen Research ref A, C, G et T: 10-1000-C2; 10-1010-C2; 10-1020-C2; 10-1030-C2 |
| 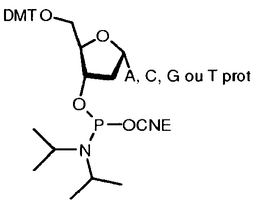 | 3'amidites-alpha | Chemgenes (A, C, G et T): ANP 1651 ; ANP 1652 ; ANP 1653 ; ANP 1654. |
| 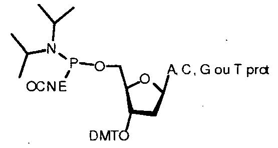 | 5'amidites-beta | Ref Glen (A, C, G et T) : 10-0001-02 ; 10-0101-02 ; 10-0201-02 ; 10-0301-02) |

***Synthèse des oligonucléotides** :* ont été utilisés pour la synthèse des oligonucléotides les 3'amidites-beta, 5'amidites-beta, 3'amidites-alpha, car leur réactivité est très similaire. Préférentiellement, il a été utilisé une CPG dabsyl et le phosphoramidite de la 6-Fluoresceine. La méthode de synthèse aux phosphoramidites a été décrite par Beaucage et Lyer, (Tetrahedron, 48, 223-2311, 1992)

D'une manière générale, le premier nucléoside de 1a séquence à synthétiser est fixé au support solide (CPG) en position 3' (ou en 5'), l'extrémité 5'OH (ou 3') du nucléoside étant protégé par un groupement acido labile diméthoxytrityl (DMT).
- Dans une première étape de détritylation, un traitement acide (acide tri ou dichloroacétique) permettait d'enlever le groupement DMT afin de générer une extrémité OH réactive.
- Dans une deuxième étape de « coupling », le phosphoramidite de la base à ajouter est condensé (en 5' ou en 3') sur ce premier site d'élongation afin de générer une liaison phosphite triester. La condensation se faisait en présence d'un catalyseur (le tétrazole ou le S-thio ethyl tétrazole, ou le DCI, ou ...)
- Dans une troisième étape de «camping » les groupements -OH qui n'auraient pas réagi lors de l'étape de condensation précédente sont bloqués par un réactif acylant (anhydride acétique) afin d'éviter des délétions dans la séquence.
- Dans une quatrième étape d'oxydation, la liaison phosphite triester était oxydée en liaison phosphate triester par un traitement oxydant (iode aqueux). La liaison phosphite triester peut également être oxydée par le réactif de Beaucage en solution dans l'acétonitrile pour donner une liaison phosphorothioate triester.

Les étapes 1 à 4 étaient répétées autant de fois qu'il était nécessaire en fonction de la longueur de la séquence à synthétiser.

Quand la séquence désirée était terminée, le support solide sur lequel se trouve l'oligonucléotide était mis a incuber dans une solution d'ammoniaque concentrés afin de cliver l'oligonucléotide du support, de déprotéger les bases et les groupements phosphates. L'oligonucléotide brut ainsi obtenu était précipité dans une solution d'acétone et de perchlorate de sodium avant d'être dosé à 260 nm (Spectramax, Molecular Device).

On purifiait alors sur colonne XTerra C8 4.6x150 3,5 µm, 50 nmole d'oligonucléotide brut en une seule fois (Chaîne HPLC Alliance, WATERS) Gradient acetonitrile/TEAAc. Les fractions les plus pures étaient contrôlées par chromatographie en échangeuse d'ion sur colonne Gen Pack Fax (Chaîne HPLC Alliance, WATERS) dans un gradient de NaCl à pH 12.

Les fractions étaient rassemblées et évaporées puis le mélange était redosé et ré-analysé en HPLC avant d'être utilisé.

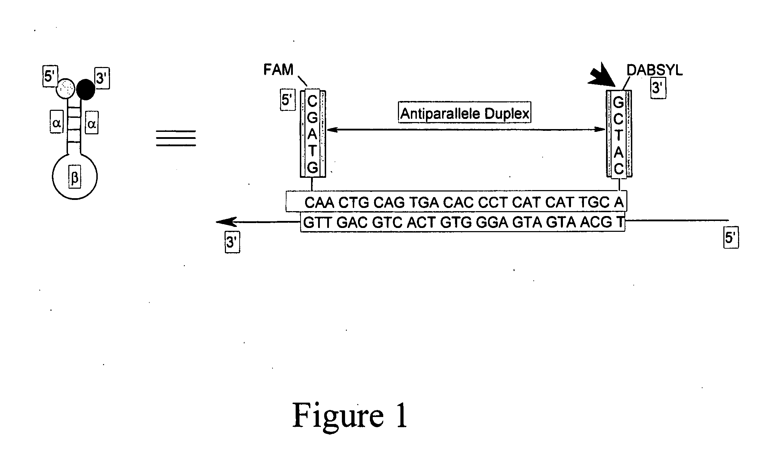

### 1A- Premier mode de réalisation de l'invention selon lequel lesdits premier et troisième segment (partie tige) de l'oligonucléotide comprennent des phosphoramidites alpha (figure 1)

Un oligonucléotide comprenant un deuxième segment ou boucle en configuration anomérique bêta, et une tige en configuration anomérique alpha (premier et troisième segment) formant un duplex antiparallèle alpha/alpha a été utilisé (oligonucléotide A). La synthèse de l'oligonucléotide selon ce premier mode de réalisation a été initiée à partir de l'extrémité comprenant le groupement dabsyl (figure 1 voir flêche). La boucle était ensuite synthétisée avec les amidites beta, puis, le troisième segment était synthétisé à partir d'amidites alpha.

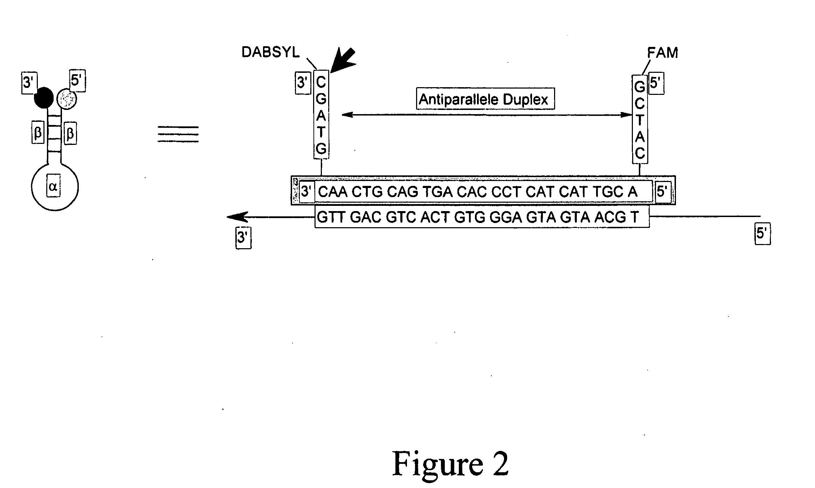

### 1B- Deuxième mode de réalisation de l'invention selon lequel ledit deuxième segment (partie «boucle ») de l'oligonucléotide comprend des phosphoramidites alpha (figure 2)

Par rapport au mode de réalisation précédent, l'oligonucléotide initial comprend ici un deuxième segment (boucle) comprenant des nucléotides alpha s'hybridant de façon parallèle avec la cible (oligonucléotide B).

Les premiers et troisième segment (partie tige) comprennent des nucléotides beta. La synthèse commençait par des amidites béta puis la boucle était synthétisée avec des amidites alpha avant de revenir aux amidite béta pour la synthèse de la tige restante (figure 2 voir flèche).

### 1C - Troisième mode de réalisation de l'invention selon lequel les dits premier, deuxième et troisième segments (parties « tige » et « boucle ») de l'oligonucléotide comprennent des phosphoramidites alpha

Par rapport au mode de réalisation précédent, l'oligonucléotide initial est ici totalement modifié avec des nucléosides alphas (boucle et tige). Les nucléotides alpha de la boucle s'hybridant de façon parallèle avec la cible tandis que ceux de la tige s'hybrident entre eux de façon anti parallèle (oligonucléotide C).

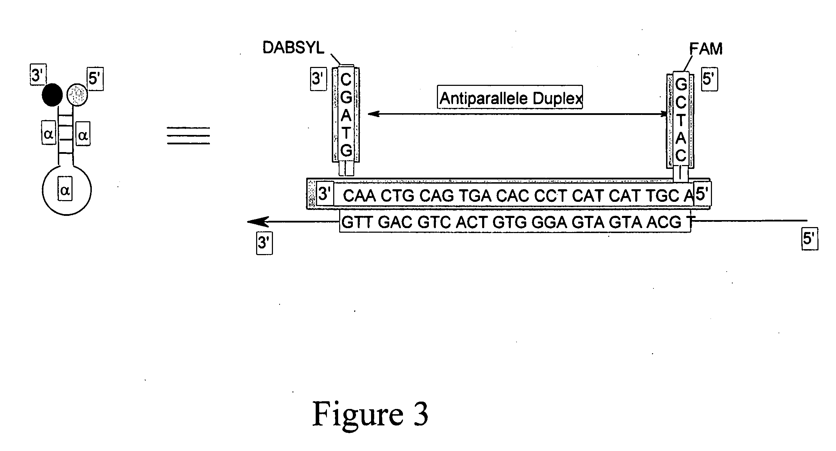

La synthèse commençait et finissait par des amidites alpha (figure 3).

### Exemple 2 : étude de la résistance des oligonucléotides selon l'invention contre le clivage par l'activité 5'-nucléase de la Taq Polymerase

L'objectif de cette expérience est de démontrer qu'un oligonucléotide modifiée selon l'invention (oligonucléotides A, B et C de l'exemple 1) n'est pas clivée par l'activité 5'-nucléase de la Taq Polymerase dans une réaction d'amplification enzymatique *in vitro.* Design expérimental: La séquence cible était le plasmide pCITE comprenant un insert de 1.2 Kb correspondant à une séquence génétique du virus hMPV, à une concentration de 5-10³ copies par tube.

Les amorces et sondes utilisées étaient les suivantes :
o Amorce sens: SEQ ID No 1 5'- CAT ATA AGC ATG CTA TAT TAA AAG AGT CTC -3'
o Amorce reverse: SEQ ID No 2 5'- CCT ATT TCT GCA GCA TAT TTG TAA TCA G -3'
o Sonde modifiée selon le premier mode de réalisation de l'invention (oligonucléotide A): SEQ ID No3 5'- FAM-[GC TAC] CAA CTG CAG TGA CAC CCT CAT CAT TGCA [GTA GC]-Dabcyl-3' - Les séquences nucléotidiques entre crochets, correspondant aux premier et troisième segments dits « tige », étaient composées de nucléotides d'anomérie alpha. La sequence soulignée, dite «boucle » d'anomerie beta reconnaissait spécifiquement unie séquence de l'amplicon généré dans l'amplification *in vitro.* FAM est un fluorophore de type fluoresceine dont la fluorescence peut être détectée à 530 nm. Dabcyl est une molécule aromatique qui empêche l'émission de fluorescence quand elle se trouve physiquement à proximité du fluorophore FAM.
o Sonde modifiée selon le deuxième mode de réalisation de l'invention (oligonucléotide B): SEQ ID No4 5'- FAM-GC TAC [ACGT TAC TAC TCC CAC AGT GAC GTC AAC] GTA GC-Dabcyl-3' - La séquence nucléotidique entre crochets, correspondant au deuxième segment dit «boucle », était composée de nucléotides d'anomerie alpha et reconnaissait spécifiquement une séquence de l'amplicon généré dans l'amplification *in vitro.*
o Sonde modifiée selon le troisième mode de réalisation de l'invention (oligonucléotide C): SEQ ID No5 5'- FAM-[GC TAC ACGT TAC TAC TCC CAC AGT GAC GTC AAC GTA GC]-Dabcyl-3' - La séquence nucléotidique entre crochets, correspondant aux premier, deuxième et troisième segments, était composée de nucléotides d'anoméiie alpha. La sequence soulignée, correspondant au deuxième segment dit « boucle », reconnaissait spécifiquement une séquence de l'amplicon généré dans l'amplification *in vitro.*
o Sonde contrôle non modifiée (MB): SEQ ID No6 5'- FAM-GC TAC CAA CTG CAG TGA CAC CCT CAT CAT TGCA GTA GC-Dabcyl -3' - Cette séquence, d'anomerie beta, reconnaissait spécifiquement une séquence de l'amplicon généré dans l'amplification *in vitro.*

### Amplification PCR avec détection en temps réel sur LightCycler:

Un kit d'amplification "LightCycler FastStart DNA Master Hybridization Probes" (Roche, Penzberg, Allemagne) a été utilisé. La préparation du mélange réactionnel était faite suivant les procédures recommandées par le fournisseur. Dans un volume réactionnel de 20 µl, on mélangeait 5·10³ copies du plasmide avec les amorces sens et reverse (0.5 µM), la sonde nucléotidique modifiée ou non modifiée (1 µM), 2 µl du vial 1 du kit (enzyme mix), 0.8 µl de MgCl₂ à 25 mM du kit et de l'eau PCR grade du kit. Le mélange réactionnel était ensuite introduit dans un tube capillaire et celui ci était introduit dans le LightCycler. Pour chaque réaction d'amplification, un témoin était fait avec la sonde non modifiée (MB) à la place de la sonde modifiée (oligonucléotide A, B ou C). Pour chaque réaction d'amplification, un témoin était également fait avec ajout d'eau PCR grade du kit à la place du plasmide cible (contrôle négatif, "c-").

La réaction PCR consistait en une dénaturation initiale de 8 minutes à 95°C, suivie de 40 cycles à 95°C pendant 30 secondes, 40°C pendant 5 secondes et 60°C pendant 60 secondes. La lecture de la fluorescence se faisait à 530 nm, en un point à la fin de chaque étape dans chaque cycle. Les résultats de fluorescence étaient ensuite retraités sur une feuille Excel de façon à séparer les résultats par température, les normaliser et en faire une représentation graphique. Les graphiques obtenus pour les lectures à 40°C et 60°C servaient à vérifier l'hybridation de la sonde avec les amplicons pendant la réaction d'amplification. A 95°C, par contre, il n'y avait pas d'hybridation possible entre la sonde et les amplicons. Toute augmentation de signal à 95°C, par rapport au contrôle négatif, ne pouvait être due qu'au clivage de la sonde. Le graphique obtenu pour la lecture à 95°C servait donc à prouver le clivage ou non de la sonde par l'activité 5'-nucléase de la TaqPol.

### Résultats:

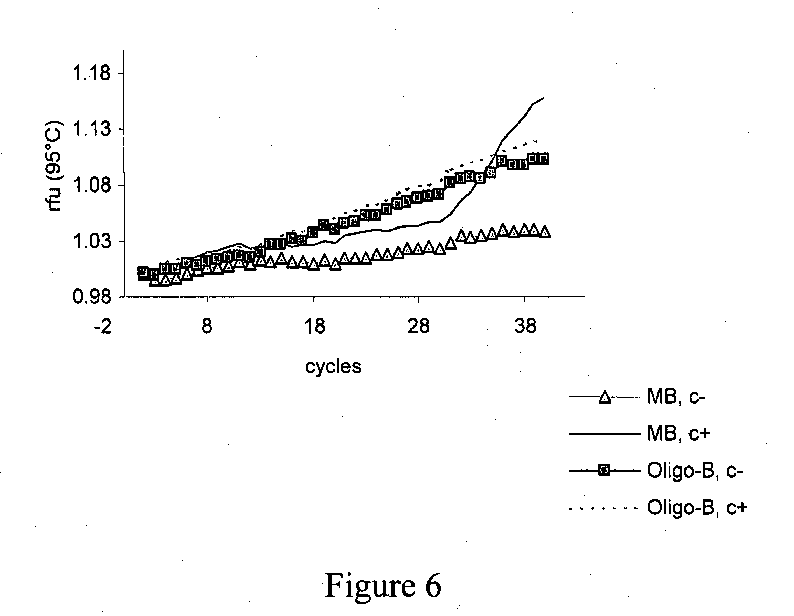

Les profils obtenus en détection en temps réel à 40°C sont présentés dans la figure 6 . L'axe horizontal représente le nombre de cycles d'amplification, l'axe vertical représente la fluorescence détectée à 530 nm, à 40°C, dans chaque cycle. En haut à gauche, «MB » ; en haut à droite, « Oligonucléotide A » ; en bas à gauche, «Oligonucléotide B » ; en bas à droite, «Oligonucléotide C». Dans chaque graphique est representé le contrôle négatif correspondant.

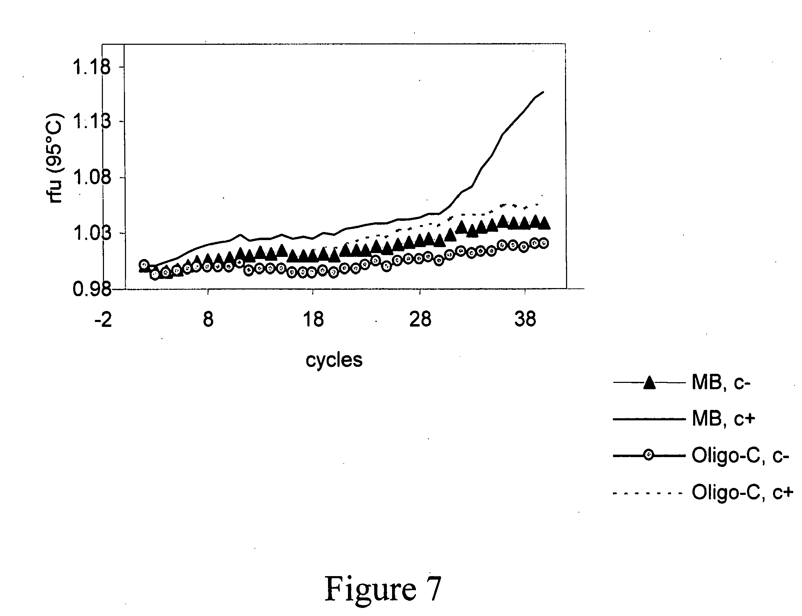

Les profils obtenus en détection en temps réel à 95°C sont présentés dans les figures 6 à 8. Dans chaque figure est représenté le profil correspondant à l'un des molecular beacons modifié, avec comme contrôle le profil du molecular beacon non modifié et celui des échantillons négatifs correspondants.

Le résultat attendu en cas d'hybridation est une augmentation exponentielle du signal de fluorescence à 40°C à partir du moment ou le nombre d'amplicons augmente. En cas de clivage, en attend une augmentation exponentielle de la fluorescence à 95°C parallèle à celle observée pour l'hybridation. Les augmentations ou diminutions linéaires du signal ne correspondent pas à une quelconque détection de l'amplicon, car celui ci est généré de façon exponentielle.

Les profils à 40°C démontraient que les sondes nucléotidiques modifiées A, B et C ainsi que le contrôle non modifié MB détectaient spécifiquement la présence des amplicons issus de la réaction enzymatique d'amplification *in vitro.*

Les profils à 95°C démontraient que les sondes MB et A étaient clivées spécifiquement pendant la réaction d'amplification alors que les sondes modifiées B et C n'étaient pas clivées dans les mêmes conditions.

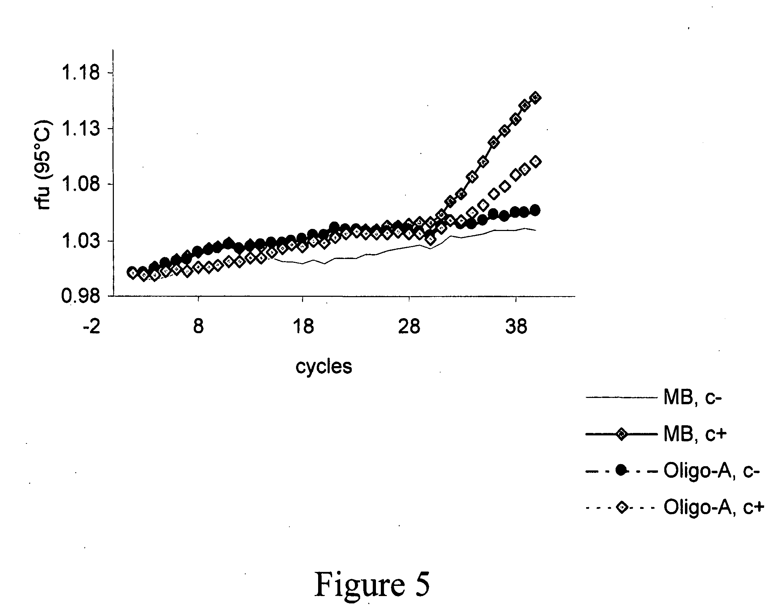

Les figures 5 à 7 présentent le comparatif des augmentations de fluorescence à 95°C pour la sonde non modifiée « MB » et, respectivement, pour la sonde modifiée « oligonucléotide A », « oligonucléotide B » et « oligonucléotide C ». On pouvait observer une augmentation spécifique de la fluorescence seulement pour la sonde MB et la sonde « oligonucleotide A », quand l'amplification a lieu. Quand l'amplification n'a pas lieu (contrôles négatifs, «c-» ) et quand le deuxième segment dit «boucle » de la sonde nucleotidique est modifié (oligonucléotides B et C), il n'y a pas de clivage par l'activité 5'-nuclease de la Taq Polymerase, et il n'y a donc pas d'augmentation de fluorescence par rapport à l'échantillon négatif.

Conclusion: Les molecular beacons modifiés dans le deuxième segment dit « boucle » selon l'invention (oligonucléotides B et C) s'hybridaient spécifiquement avec les amplicons issus du cyclage sur lightcycler, donnant lieu à une détection en temps réel comparable à celle obtenue avec le molecular beacon non modifié « MB » et avec le molecular beacon modifié dans la tige (oligonucléotide A). Les molecular beacons modifiés dans la boucle n'étaient pas clivés par l'activité 5'-nuclease de la Taq Polymerase, contrairement au molecular beacon contrôle « MB » et au molecular beacon modifié sur la tige « oligonucléotides A ».

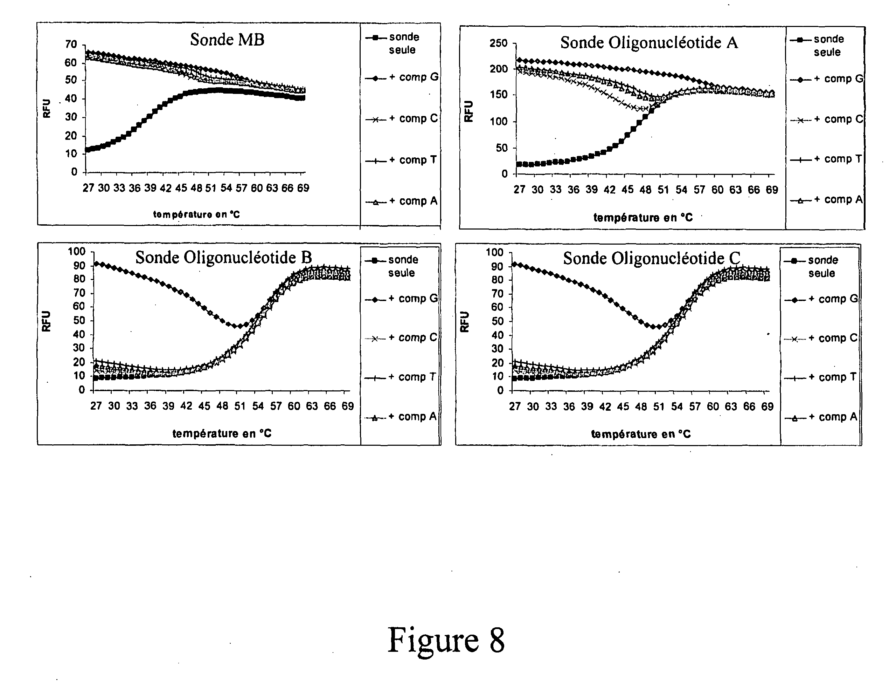

### Exemple 3: étude de la spécificité de l'hybridation à leur séquence cible des oligonucléotides selon l'invention (figure 8)

### Obiectif :

Le but de l'expérience est de vérifier si des beacons comportant des nucléotides de type alpha sont plus spécifiques pour leur cible que des beacons composés uniquement de nucléotides de type bêta.

La spécificité peut être mesurée par le calcul de la différence de Tm (ici ΔTm) entre une sonde en solution avec sa cible parfaitement complémentaire et cette sonde en solution avec une cible comportant une variation d'un nucléotide dans sa séquence (ici mismatch). Les ΔTm seront calculés pour chaque sonde et pour chaque séquence complémentaire étudiée.

Dans notre cas la spécificité sera mesurée par la valeur des ΔTm calculés avec les sondes Oligonucléotide A, B et C et quatre séquences complémentaires dont une est parfaitement complémentaire du deuxième segment des sondes et les trois autres comportent un mismatch C, T ou A par rapport au complémentaire parfait.

### Design expérimental

Les sequences des sondes et des complémentaires utilisés sont décrites dans le tableau 1 ci dessous.

La fluorescence de solutions contenant les sondes, en présence de cible parfaitement complémentaire ou avec un mismatch, a été mesurée en fonction de la température entre 69 et 27°C dans un spectrofluorimètre thermoanalyseur EasyQ (bioMérieux bv, Boxtel, NL).. Toutes les mesures ont été effectuées sur des solutions de 20µL de volume à une concentration de la sonde de 100nM, et à la concentration en cible de 1µM, dans une solution aqueuse tamponnée du Nasba Basic kit (bioMérieux bv, Boxtel, NL).

**Tableau 1: Séquences des sondes et complémentaires utilisés. En italique gras : nucléotides alpha: Souligné: mismatch. Toutes les séquences sont écrites dans le sens 5'-3'.**

| SEQ ID | Noms | Séquences |
|---|---|---|
| 9 | MB | fam-CGA TG-C AAC TGC AGT GAC ACC CTC ATC ATT GCA-CAT CG-dabsyl |
| 10 | Oligo A | fam*-**CGA TG***-C AAC TGC AGT GAC ACC CTC ATC ATT GCA-***CAT CG**-*dabsyl |
| 11 | Oligo B | fam-GCT AC-***ACG TTA CTA CTC CCA CAG TGA CGT CAA C*** -GT AGC-dabsyl |
| 12 | Oligo C | fam-***GCT AC-ACG TTA CTA CTC CCA CAG TGA CGT CAA C-GT ACG**-*clabsyl |
| 13 | comp G | TT ATG ATG AGG GTG TCA CTG CAT T |
| 14 | comp C | TT ATG ATG AGG CTG TCA CTG CAT T |
| 15 | comp T | TT ATG ATG AGG TTG TCA CTG CAT T |
| 16 | comp A | TT ATG ATG AGG ATG TCA CTG CAT T |

### Résultats

Les profils de dénaturation thermique obtenus sont représentés dans la figure 8. La Tm correspond au maximum de la première dérivée de chaque profil. Les valeurs de Tm obtenus sont indiqués dans le tableau 2 ci dessous.

**Tableau 2: Tm des différentes sondes avec les complémentaires G, C. T et A. Entre parenthèses, valeurs de ΔTm entre le complémentaire parfait « comp G » et les autres complémentaires contenant un mismatch.**

| Tm en °C | MB | Oligonucléotide A | Oligonucléotide | B Oligonucléotide C |
|---|---|---|---|---|
| comp G | 56,9 | 56,4 | 44,3 | 42,3 |
| comp C | 44,9 (-12,0) | 41,8 (-14,6) | <30 | <30 |
| comp T | 47,7 (-9,2) | 45,4 (-11,0) | <30 | <30 |
| comp A | 46,6 (-10,3) | 43,9 (-12,5) | <30 | <30 |

Dans les cas de la sonde Oligonucleotide A, les valeurs de ΔTm dans le tableau 2 montrent une meilleure spécificité que la sonde MB, autour de +2°C. Etant donné que dans les deux cas la séquence complémentaire est de type bêta, cette augmentation de la spécificité est certainement due à une plus grande stabilité de la hybridation de la tige (premier et troisième segments complémentaires) dans le cas de la sonde Oligonucléotide A, qui peut être observée dans la figure 14.

Dans le cas des sondes Oligonucléotide B et C, les valeurs de Tm avec les séquences complémentaires contenant un mismatch n'ont pas pu être obtenus car l'hybridation n'était pas suffisante dans la plage de températures utilisée. Ceci indique une très grande spécificité, qui peut être vérifié dans les profils de dénaturation thermique dans la figure 8. On peut voir dans ces profils que seul le complémentaire parfait « comp G » s'hybride avec ces sondes, pendant que les complémentaires contenant un mismatch ne s'hybrident pas. Par rapport aux résultats obtenus avec la sonde Oligonucléotide A, ces résultats montrent que la augmentation de la spécificité due à l'hybridation d'une séquence de type alpha est indépendante de la nature de la tige, qui est de type bêta dans la sonde Oligonucléotide B et de type alpha dans la sonde Oligonucléotide C.

### Conclusion

Les résultats montrent que les quatre sondes modifiées possèdent une spécificité plus importante que la sonde non modifiée MB (tableau 2). Cette augmentation de la spécificité est indépendante dans notre cas de la nature de la tige (premier et troisième segments complémentaires).

### Exemple 4: Etude de l'hybridation de la tige d'une sonde nucléique de type molecular beacon avec une séquence cible

### Objectifs

Lors de l'hybridation d'une sonde nucléique de type molecular beacon avec sa séquence cible complémentaire, il est possible que la tige de la sonde, autant du côté 5' que 3', puise aussi s'hybrider avec la cible. Cela peut produire une augmentation non prévue de la stabilité de l'hybridation et par conséquence une perte de la spécificité de la sonde. L'exemple ici décrit sert à montrer que la combinaison de nucléotides alpha et bêta dans une sonde nucléique de type molecular beacon permet d'éviter ce problème en empêchant l'hybridation de la tige avec la cible complémentaire.

### Design expérimental

Les séquences des sondes utilisées sont les mêmes que dans l'exemple 3, et sont décrites dans le tableau 1 plus haut. Les séquences complémentaires utilisées sont décrites dans le tableau 3 ci dessous.

**Tableau 3: Séquences des oligonucléotitles cible complémentaires utilisés. En gras, la séquence complémentaire de la boucle de la sonde.**

| Noms | Séquences |
|---|---|
| SEQ ID No18: | |
| comp+ | 5'-CGA **TG T TCA ATG ATG AGG GTG TCA CTG CA**T T |
| SEQ ID No19: | |
| comp- | 5'-GTA GC**T GCA ATG ATG AGG GTG TCA CTG CA**T T |

Les séquences complémentaires comportent une partie du côté 3' qui est complémentaire de la boucle de la sonde, et une partie du côté 5' qui est complémentaire, dans le cas de « comp+ », ou pas dans le cas de « comp-» d'un côté de la tige de la sonde.

La fluorescence de solutions contenant les différentes combinaisons de sondes et de cibles a été mesurée en fonction de la température entre 69 et 27°C dans un spectrofluorimètre thermoanalyseur EasyQ (bioMérieux bv, Boxtel, NL).. Toutes les mesures ont été effectuées sur des solutions de 20µL de volume à une concentration de la sonde de 100nM, et à la concentration en cible de 1µM, dans une solution aqueuse tamponnée du Nasba Basic kit (bioMérieux bv, Boxtel, NL).

### Résultats

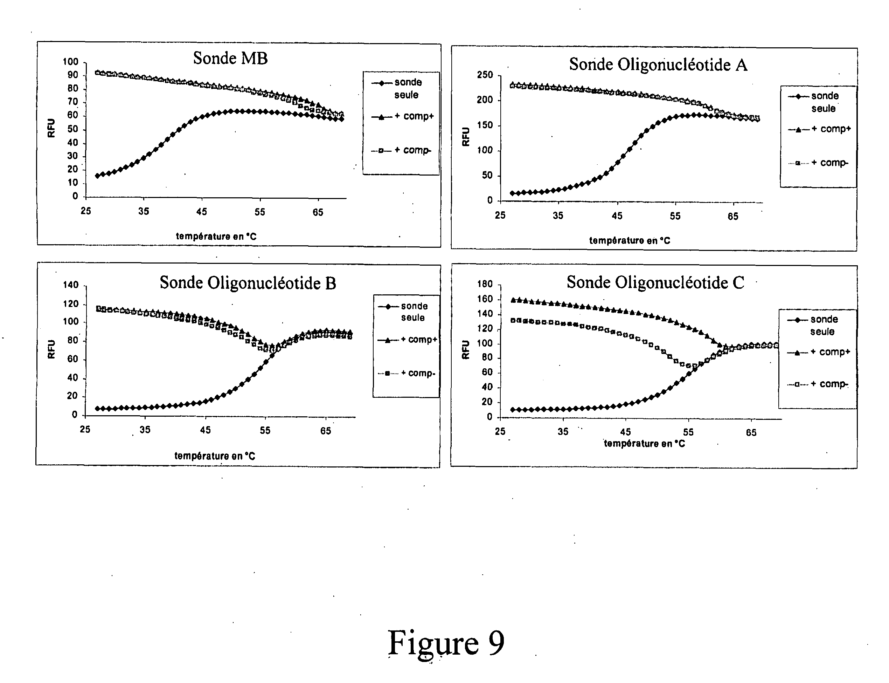

Les profils de dénaturation thermique obtenus sont représentés dans la figure 9. La Tm correspond au maximum de la première dérivée de chaque profil. Les valeurs de Tm obtenus sont indiqués dans le tableau 4 ci dessous.

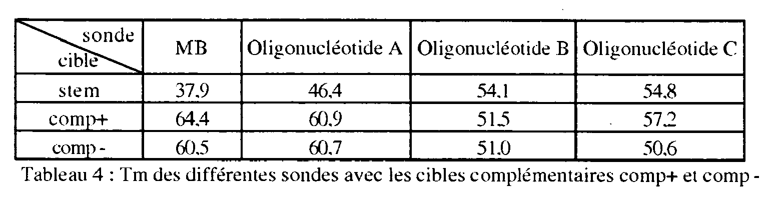

Une sonde dont la boucle est de type bêta s'hybridera en orientation antiparallèle sur une séquence cible naturelle, forcement de type bêta, comme celles utilisées ici. Une sonde dont la boucle est de type alpha s'hybridera en orientation parallèle sur les mêmes séquences cible bêta. De ce fait, en fonction de la nature de la boucle, la tige va se retrouver face à la séquence de la cible dans l'orientation dictée par l'hybridation de la boucle. La tige alpha d'une sonde ne peut donc s'hybrider sur la cible naturelle bêta que si celle ci est complémentaire (comp+) et que la boucle de la sonde est aussi alpha (orientation parallèle). De la même façon, la tige bêta d'une sonde ne peut s'hybrider sur la cible naturelle bêta que si celle si est complémentaire (comp+) et que la boucle de la sonde est aussi bêta (orientation antiparallèle). Ceci implique que la tige des sondes MB et Oligonucléotide C peuvent s'hybrider avec la séquence complémentaire de comp+, mais pas avec la séquence non complémentaire de comp- provocant une différence de Tm, qu'on peut observer dans le tableau 4. La tige des sondes Oligonucléotide A et B ne peuvent pas s'hybrider avec la séquence complémentaire de comp+ ni avec la séquence non complémentaire de comp-, et la Tm est similaire avec les deux cibles, comme on peut voir dans le tableau 4.

### Conclusion

Les résultats montrent que les sondes nucléiques de type molecular beacon dont les nucléotides de la tige sont de anomérie différente que les nucléotides de la boucle (sondes Oligonucléotide A et B) empêchent l'hybridation de la tige avec la séquence complémentaire présente dans la cible, même quand la complémentaiité est parfaite. Aucun effet négatif sur l'hybridation n'est observé.

## Revendications

1. Oligonucléotide marqué comprenant un premier segment nucléotidique, un deuxième segment nucléotidique, complémentaire d'une séquence cible, et un troisième segment nucléotidique, complémentaire dudit premier segment nucléotidique, **caractérisé en ce qu'**il comprend un fluorophore, un extincteur et au moins un nucléoside d'anomérie alpha

2. Oligonucléotide marqué selon la revendication 1 **caractérisé en ce que** lesdits premier et troisième segments sont de part et d'autre du deuxième segment.

3. Oligonucléotide marqué selon la revendication 1 ou 2 **caractérisé en ce que** ledit deuxième segment comprend au moins un nucléoside d'anomérie alpha.

4. Oligonucléotide marqué selon l'une quelconque des revendications 1 à 3 **caractérisé en ce que** le fluorophore est à une extrémité dudit oligonucléotide et l'extincteur est à l'autre extrémité dudit oligonucléotide.

5. Oligonucléotide marqué selon l'une quelconque des revendications 1 à 4 **caractérisé en ce que** ledit fluorophore est une fluoresceine.

6. Oligonucléotide marqué selon l'une quelconque des revendications 1 à 5 **caractérisé en ce que** ledit extincteur est le dabsyl.

7. Oligonucléotide marqué selon l'une quelconque des revendications 1 à 6 **caractérisé en ce que** ledit premier segment comprend de 3 à 8 nucléotides, ledit deuxième segment comprend de 10 à 35 nucléotides, et ledit troisième segment, lorsqu'il est présent comprend de 3 à 8 nucléotides.

8. Procédé pour détecter un matériel nucléique dans un échantillon biologique comprenant les étapes suivantes :
a) on extrait le matériel nucléique d'un échantillon biologique,
b) on amplifie le matériel nucléique pour obtenir des amplicons d'au moins une séquence cible du matériel nucléique
c) on utilise, simultanément à l'étape b) ou postérieurement à l'étape b), au moins un oligonucléotide marqué tel que revendiqué dans les revendications 1 à 7
d) on détecte la présence desdits amplicons

## Claims

1. Labeled oligonucleotide comprising a first nucleotide segment, a second nucleotide segment, complementary to a target sequence, and a third nucleotide segment, complementary to said first nucleotide segment, **characterized in that** it comprises a fluorophore, a quencher and at least one alpha-anomeric nucleoside.

2. Labeled oligonucleotide according to Claim 1, **characterized in that** said first and third segments are on either side of the second segment.

3. Labeled oligonucleotide according to Claim 1 or 2, **characterized in that** said second segment comprises at least one alpha-anomeric nucleoside.

4. Labeled oligonucleotide according to any one of Claims 1 to 3, **characterized in that** the fluorophore is at one end of said oligonucleotide and the quencher is at the other end of said oligonucleotide.

5. Labeled oligonucleotide according to any one of Claims 1 to 4, **characterized in that** said fluorophore is fluorescein.

6. Labeled oligonucleotide according to any one of Claims 1 to 5, **characterized in that** said quencher is dabsyl.

7. Labeled oligonucleotide according to any one of Claims 1 to 6, **characterized in that** said first segment comprises from 3 to 8 nucleotides, said second segment comprises from 10 to 35 nucleotides, and said third segment, when it is present, comprises from 3 to 8 nucleotides.

8. Method for detecting a nucleic material in a biological sample, comprising the following steps:
a) the nucleic material is extracted from a biological sample,
b) the nucleic material is amplified in order to obtain amplicons of at least one target sequence of the nucleic material,
c) at least one labeled oligonucleotide according to Claims 1 to 7 is used simultaneously with step b) or subsequent to step b),
d) the presence of said amplicons is detected.

## Patentansprüche

1. Markiertes Oligonukleotid, umfassend einen ersten Nukleotidabschnitt, einen zweiten Nukleotidabschnitt, der zu einer Zielsequenz komplementär ist, und einen dritten Nukleotidabschnitt, der zu diesem ersten Nukleotidabschnitt komplementär ist, **dadurch gekennzeichnet, dass** es einen Fluorophor, einen Quencher und mindestens ein alpha-anomeres Nukleosid umfasst.

2. Markiertes Oligonukleotid nach Anspruch 1, **dadurch gekennzeichnet, dass** der erste und der dritte Abschnitt von beiden Seiten des zweiten Abschnitts stammen.

3. Markiertes Oligonukleotid nach Anspruch 1 oder 2, **dadurch gekennzeichnet, dass** der zweite Abschnitt mindestens ein alpha-anomeres Nukleosid umfasst.

4. Markiertes Oligonukleotid nach einem der Ansprüche 1 bis 3, **dadurch gekennzeichnet, dass** sich der Fluorophor an einem Ende des Oligonukleotids und der Quencher am anderen Ende des Oligonukleotids befindet.

5. Markiertes Oligonukleotid nach einem der Ansprüche 1 bis 4, **dadurch gekennzeichnet, dass** es sich bei dem Fluorophor um ein Fluorescein handelt.

6. Markiertes Oligonukleotid nach einem der Ansprüche 1 bis 5, **dadurch gekennzeichnet, dass** es sich bei dem Quencher um Dabsyl handelt.

7. Markiertes Oligonukleotid nach einem der Ansprüche 1 bis 6, **dadurch gekennzeichnet, dass** der erste Abschnitt 3 bis 8 Nukleotide umfasst, der zweite Abschnitt 10 bis 35 Nukleotide umfasst und der dritte Abschnitt, wenn er vorhanden ist, 3 bis 8 Nukleotide umfasst.

8. Verfahren zum Nachweisen von Nukleinsäurematerial in einer biologischen Probe, umfassend die folgenden Schritte:
a) Extrahieren des Nukleinsäurematerials aus einer biologischen Probe,
b) Amplifizieren des Nukleinsäurematerials, um zu Amplicons von mindestens einer Zielsequenz des Nukleinsäurematerials zu gelangen,
c) gleichzeitig mit Schritt b) bzw. nach Schritt b) Verwenden von mindestens einem markierten Oligonukleotid nach einem der Ansprüche 1 bis 7,
d) Nachweisen des Vorhandenseins dieser Amplicons.
